# FASCICULE DE BREVET EUROPEEN

(11) **EP 4 228 739 B1**
(45) Date de publication et mention de la délivrance du brevet: **06.11.2024**
(21) Numéro de dépôt: 21787483.3
(22) Date de dépôt: 15.10.2021
(51) Int. Cl.: A61N 1/05

(54) **ENSEMBLE D'UN ACCESSOIRE D'IMPLANTATION ET D'UNE SONDE FLEXIBLE IMPLANTABLE DE STIMULATION**
SET AUS EINEM IMPLANTATIONSZUBEHÖR UND EINER IMPLANTIERBAREN FLEXIBLEN STIMULATIONSSONDE
SET OF AN IMPLANTATION ACCESSORY AND A FLEXIBLE IMPLANTABLE PACING LEAD

(30) Priorité: 15.10.2020 FR 2010566
(43) Date de publication de la demande: 23.08.2023
(73) Titulaire: Sorin CRM SAS, 92140 Clamart (FR)
(72) Inventeur: AMARO, Diego, 92350 Le Plessis-Robinson (FR); OLLIVIER, Jean-Francois, 91190 Gif-sur-Yvette (FR)
(74) Mandataire: Ungerer, Olaf
(86) Numéro de dépôt international: PCT/EP2021/078641
(87) Numéro de publication internationale: WO 2022/079257

(56) Documents cités:
- EP-A1- 0 784 993
- EP-A1- 0 819 445
- US-A1- 2005 136 385
- US-A1- 2006 041 300
- US-A1- 2014 107 756
- US-A1- 2015 157 268
- US-A1- 2015 320 330
- US-A1- 2019 038 893

## Description

### Domaine technique

La présente invention se rapporte à un ensemble d'un accessoire d'implantation et d'une sonde flexible implantable de stimulation pour un dispositif médical implantable actif.

### Arrière-plan technique

Il est connu de stimuler le ventricule droit en logeant une sonde dite endocardiaque dans le ventricule droit du coeur où elle y est introduite via le réseau veineux, ou au moyen d'une sonde dite endocavitaire implantée dans les cavités via un accès veineux.

Il est également connu d'utiliser des sondes dites épicardiques qui sont directement fixées sur la paroi extérieure du myocarde. Cependant, une sonde épicardique fournit souvent des résultats moins bons que ceux obtenus au moyen d'une sonde endocardiaque, en particulier au niveau des performances électriques.

De plus, il est connu de maintenir les sondes épicardiques à la paroi de l'épicarde par suturation ou vissage d'une vis hélicoïdale d'ancrage de la sonde, mais ces fixations mécaniques ont le désavantage d'être invasives et relativement traumatiques pour les tissus.

Ainsi, l'inconvénient le plus portant des sondes épicardiques connues est le côté invasif de l'intervention par rapport aux sondes endocavitaires.

De plus, outre le caractère invasif de leur implantation, un autre inconvénient commun à ces sondes épicardiques connues, est leur faible performance électrique, ce qui va au détriment de l'efficacité de la stimulation.

Le US-A1-2005/136385 divulgue un appareil de gestion du rythme cardiaque comprenant un boîtier proximal, un boîtier distal et un fil. Le boîtier proximal comprend un premier dispositif de stockage d'énergie. Le module distal est implantable dans le coeur d'un patient et comprend un deuxième dispositif de stockage d'énergie, au moins une électrode et un module de commande. Le module de commande contrôle la délivrance d'au moins un stimulus électrique depuis le deuxième dispositif de stockage d'énergie vers un emplacement en communication avec le coeur du patient. Le fil connecte le boîtier proximal au module distal et est configuré pour communiquer un ou plusieurs signaux numériques entre le boîtier proximal et le module distal.

Le US-A1-2015/320330 divulgue des appareils de détection/stimulation électriques permettant de positionner au moins une électrode dans un tissu corporel. Un appareil de détection/stimulation électrique peut comprendre un corps de conducteur allongé ayant au moins une lumière interne, au moins une électrode de détection/stimulation, un élément de déplacement déployable/rétractable qui déplace ou sollicite au moins une électrode vers une direction présente par l'utilisateur, un mécanisme de fixation de tissu pour fixer le segment distal du dispositif au tissu corporel, et une terminaison de corps de sonde distale atraumatique.

Le US-A1-2015/157268 divulgue des systèmes, des dispositifs et des procédés pour ancrer des implants dans le corps humain, et plus spécifiquement des moyens de rétention pour fixer dans une chambre cardiaque un implant sensoriel comprenant un corps allongé renfermant une lumière et un élément sensoriel disposé à l'intérieur, un élément de retenue proximal couplé audit corps allongé et comprenant une pluralité de saillies, et un élément de retenue distal positionné de manière distale par rapport audit élément de retenue proximal et couplé audit corps allongé.

### Description de l'invention

L'invention est définie dans la revendication 1. D'autres aspects et modes de réalisation préférés sont définis dans les revendications annexées. Les aspects, les réalisations et les exemples de la présente divulgation qui n'entrent pas dans le cadre des revendications annexées ne font pas partie de l'invention et sont simplement fournis à titre d'illustration.

L'invention vise à s'affranchir des diverses limitations exposées ci-dessus, en proposant une sonde de stimulation permettant d'améliorer l'efficacité de la stimulation tout en réduisant le caractère invasif de son implantation.

L'objet de la présente invention est atteint au moyen d'un ensemble d'un accessoire d'implantation et d'une sonde flexible implantable de stimulation. Ladite sonde comprend un corps de sonde, et est apte à être combinée à un dispositif médical implantable actif via son extrémité proximale et est configurée pour être implantée à travers une paroi du coeur, en particulier à travers la paroi libre du ventricule droit, via son extrémité distale. L'accessoire d'implantation comprend une aiguille avec une extrémité libre pointue de ponction, l'aiguille étant au moins dans sa partie distale une aiguille creuse comprenant une lumière interne débouchante sur l'extrémité libre pointue de ponction, au moins une première portion de la sonde étant apte à être insérée via son extrémité distale dans la lumière interne de l'aiguille. Dans un état où la première portion de la sonde est insérée dans la lumière de l'aiguille, ladite première portion de la sonde comprend au moins une première branche qui s'étend à partir du corps de sonde dans une direction orientée vers l'extrémité libre pointue de l'aiguille, la première branche s'étendant à partir du corps de sonde depuis un point de jonction disposé à une distance prédéterminée de l'extrémité distale formant une deuxième portion du corps de sonde entre le point de jonction de la première branche et l'extrémité distale de la sonde.

Ainsi, l'aiguille permet de ponctionner la paroi du ventricule droit du coeur et d'y introduire la sonde. La sonde comprenant au niveau de son extrémité distale une première branche et une deuxième portion du corps de sonde, ladite sonde est ainsi pourvue d'un moyen de retenue lorsque la sonde est implantée à travers la paroi libre du ventricule droit. Ce moyen de retenue de la sonde est insérable dans l'aiguille au moyen de laquelle il peut être implanté dans les tissus cardiaques.

La sonde étant implantable à travers la paroi libre du ventricule droit la première branche et la deuxième portion du corps de sonde peuvent être disposées dans le ventricule droit, en particulier contre la paroi interne.

La présente invention, relative à un ensemble d'un accessoire d'implantation et d'une sonde flexible implantable de stimulation, peut être davantage améliorée grâce aux modes de réalisation suivants.

Le corps de sonde comprend en outre une troisième portion moins rigide que ladite première branche et la deuxième portion, la troisième portion s'étendant vers l'extrémité proximale de la sonde depuis le point de jonction de la première branche, de manière à ce que la première branche et la deuxième portion soient configurées pour pivoter solidairement l'une de l'autre par rapport audit point de jonction.

Ainsi, la première branche et de la deuxième portion sont rendues solidaires l'une de l'autre lors d'un mouvement de pivotement autour du point de jonction. Ce pivotement permet de disposer le moyen de retenue de la sonde contre la paroi interne du ventricule droit dans un état implanté de la sonde.

Selon un mode de réalisation, dans un état où la première portion de la sonde est insérée dans la lumière de l'aiguille, la première branche et la deuxième portion peuvent former un angle obtus et la première branche et la troisième portion peuvent former un angle aigu.

Ainsi, la première branche est arrangée de manière à pouvoir fournir un moyen de retenue dans un état implanté de la sonde suite au pivotement solidaire de la première branche et de la deuxième portion par rapport au point de jonction.

Selon un mode de réalisation, la première branche ou/et la deuxième portion peut comprendre au moins une électrode.

Ainsi, dans un état implanté de la sonde, la au moins une électrode, en particulier l'anode, peut être disposée sur la paroi interne du ventricule droit. De ce fait, grâce au contact direct de l'anode avec la paroi cardiaque, la stimulation peut être améliorée.

Selon un mode de réalisation, le corps de sonde peut comprendre au moins une électrode qui est espacée du point de jonction de la sonde par une longueur comprise entre 2 et 50 mm, en particulier entre 2 et 30 mm.

Ainsi, ces dimensions permettent, dans un état implanté de la sonde, de disposer la au moins une électrode, en particulier la cathode, dans l'épaisseur de la paroi cardiaque, notamment de la paroi libre du ventricule droit. Ainsi, la stimulation et la détection est d'autant plus améliorée. Il est aussi rendu possible de stimuler à des énergies plus basses que lorsque les électrodes ne sont pas en contact direct avec la paroi cardiaque.

Selon un mode de réalisation, la première branche et la deuxième portion peuvent avoir chacune une longueur comprise entre 2 et 20 mm.

Ces dimensions permettent d'améliorer la surface de contact du moyen de retenue contre la paroi interne du ventricule droit et ainsi d'augmenter la retenue de la sonde à travers la paroi interne du ventricule droit, tout en permettant un retrait simple et sans dommage du dispositif par traction soutenue.

Selon un mode de réalisation, le corps de sonde peut comprendre une zone renforcée et élastique par rapport au reste du corps de sonde, ladite zone étant distincte de la première portion et correspondant à une zone de pliure de la sonde.

Ainsi, la robustesse, et donc la durée de vie de la sonde, peuvent être augmentées malgré le pliage de la sonde à cet endroit.

De plus, l'élasticité de cette zone de pliure permet un déploiement élastique automatique et donc simple à mettre en oeuvre, dès lors que la première portion de la sonde est déplacée hors de la lumière de l'aiguille et qu'elle n'est plus retenue contre la paroi interne de la lumière de l'aiguille.

Selon un mode de réalisation, la première portion de la sonde peut comprendre en outre un moyen d'appui formé par une deuxième branche qui s'étend à partir du corps de sonde dans une direction orientée vers l'extrémité distale de la sonde, la deuxième branche s'étendant à partir du corps de sonde depuis un point de jonction du corps de sonde distinct de ladite deuxième portion et de la première branche.

Le moyen d'appui permet d'améliorer le maintien de la sonde à la paroi du coeur en empêchant une migration involontaire de la sonde dans le ventricule après implantation de ladite sonde. Le moyen d'appui prend en effet appui contre la paroi externe libre du ventricule droit dans l'état implanté de ladite sonde.

Selon un mode de réalisation, le point de jonction de la deuxième branche avec le corps de sonde peut être espacé d'une distance comprise entre 1 à 30 mm du point de jonction de la première branche.

Ces dimensions correspondent essentiellement à l'épaisseur de la paroi cardiaque au niveau du ventricule droit. Ainsi, la sonde est structurellement configurée pour que la première branche et la deuxième branche fassent saillie depuis le corps de sonde de part et d'autre de la paroi libre du ventricule droit dans un état implanté de la sonde, chacune des branches permettant de retenir la sonde au muscle cardiaque.

Selon un mode de réalisation, le corps de sonde peut comprendre au moins une électrode disposée entre le point de jonction de la première branche et le point de jonction de la deuxième branche.

Ainsi, la au moins une électrode peut être avantageusement disposée dans l'épaisseur de la paroi libre du ventricule droit, ce qui améliore la stimulation du ventricule droit.

Selon un mode de réalisation, la sonde peut être un microcâble flexible comprenant un coeur électriquement conducteur revêtu d'une couche électriquement isolante, et la au moins une électrode étant formée par une zone dénudée du microcâble et le diamètre du microcâble étant d'au plus 1 French (0,33mm).

Ainsi, grâce aux dimensions du microcâble, il est possible de rendre l'implantation de la sonde moins invasive. En effet, la ponction à travers les tissus cardiaques pour introduire le microcâble peut être réduite à un diamètre d'environ 1 French (0.33 mm). De ce fait, il est rendu possible de moins endommager les tissus lors de l'implantation et du retrait de la sonde.

L'objet de l'invention est également atteint au moyen d'une sonde flexible implantable de stimulation pour un ensemble tel que décrit ci-dessus. Ladite sonde comprend un corps de sonde. Ladite sonde est apte à être combinée à un dispositif médical implantable actif via son extrémité proximale et étant configurée pour être implantée à travers une paroi du coeur, en particulier à travers la paroi libre du ventricule droit, via son extrémité distale. Ladite sonde comprend une première portion configurée pour être insérée dans une aiguille. La première portion comprend au moins une première branche qui s'étend à partir du corps de sonde dans une direction orientée vers l'extrémité proximale de la sonde et vers l'extrémité libre pointue de l'aiguille lorsque la première portion est insérée dans l'aiguille, la première branche s'étendant à partir du corps de sonde depuis une distance prédéterminée de l'extrémité distale formant une deuxième portion du corps de sonde entre la première branche et l'extrémité distale de la sonde.

La sonde comprenant au niveau de son extrémité distale une première branche et une deuxième portion du corps de sonde, ladite sonde est ainsi pourvue d'un moyen de retenue lorsque la sonde est implantée à travers la paroi libre du ventricule droit. Ce moyen de retenue de la sonde est insérable dans l'aiguille au moyen de laquelle il peut être implanté dans les tissus cardiaques.

La sonde étant implantable à travers la paroi libre du ventricule droit, de sorte que la première branche et la deuxième portion du corps de sonde soient disposées dans le ventricule droit, contre la paroi interne, les zones de contact entre la sonde et la paroi du myocarde sont augmentées, ce qui permet d'améliorer les performances électriques de la sonde.

Selon un mode de réalisation, la première portion de la sonde peut comprendre en outre un moyen d'appui formé par une deuxième branche qui s'étend à partir du corps de sonde dans une direction orientée vers l'extrémité distale de la sonde, la deuxième branche s'étendant à partir du corps de sonde depuis un point de jonction du corps de sonde à la fois distinct de ladite deuxième portion et de la première branche.

Le moyen d'appui permet d'améliorer le maintien de la sonde à la paroi du coeur en empêchant une migration involontaire de la sonde dans le ventricule après implantation de ladite sonde. Le moyen d'appui prend en effet appui contre la paroi externe libre du ventricule droit dans l'état implanté de ladite sonde.

### Description des figures

L'invention et ses avantages seront expliqués plus en détail dans ce qui suit au moyen de modes de réalisation préférés et en s'appuyant notamment sur les figures d'accompagnement suivantes, dans laquelle :
La Figure 1a représente une vue schématique et partielle de l'ensemble d'un accessoire d'implantation et d'une sonde flexible implantable de stimulation selon la présente invention dans un état non implanté de la sonde.
La Figure 1b représente une vue schématique et partielle de la sonde flexible implantable de stimulation illustrée à la Figure 1a dans un état non implanté de la sonde.
La Figure 1c représente une vue schématique de la sonde flexible implantable de stimulation illustrées aux Figures 1a et 1b dans un état implanté de la sonde.
La Figure 1d représente une vue schématique et partielle de la sonde flexible implantable de stimulation illustrées aux Figures 1a à 1c selon une première variante.
La Figure 1e représente une vue schématique et partielle de la sonde flexible implantable de stimulation illustrées aux Figures 1a à 1c selon une deuxième variante.
La Figure 1f représente une vue schématique et partielle de la sonde flexible implantable de stimulation illustrées aux Figures 1a à 1c selon une troisième variante.
La Figure 2a représente une vue schématique d'une étape de l'implantation de la sonde selon la présente invention.
La Figure 2b représente une vue schématique d'une étape de l'implantation de la sonde selon la présente invention.
La Figure 2c représente une vue schématique d'une étape de l'implantation de la sonde selon la présente invention.
La Figure 2d représente une vue schématique d'une étape de l'implantation de la sonde selon la présente invention.
La Figure 2e représente une vue schématique d'une étape de l'implantation de la sonde selon la présente invention.
La Figure 2f représente une vue schématique d'une étape de l'implantation de la sonde selon la présente invention.
La Figure 2g représente une vue schématique d'une étape de l'implantation de la sonde selon la présente invention.
La Figure 2h représente une vue schématique d'une étape de l'implantation de la sonde selon la présente invention.
La Figure 2i représente une vue schématique d'une étape de l'implantation de la sonde selon la présente invention.
La Figure 2j représente une vue schématique d'une étape de l'implantation de la sonde selon la présente invention.
La Figure 2k représente une vue schématique d'une étape de l'implantation de la sonde selon la présente invention.

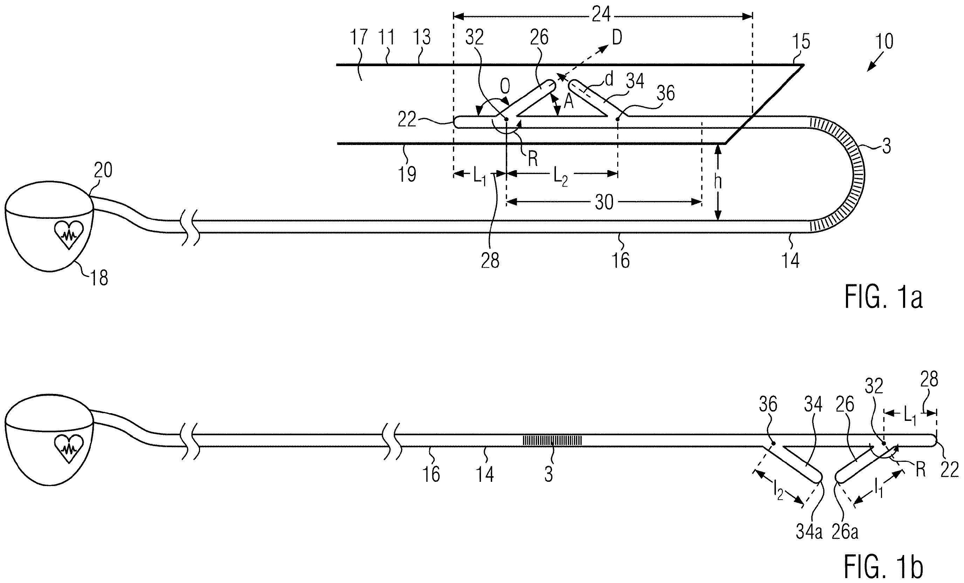

La **Figure 1a** représente une vue schématique et partielle de l'ensemble 10 d'un accessoire d'implantation 11 et d'une sonde 14 flexible implantable de stimulation selon la présente invention dans un état non implanté de la sonde. Par soucis de clarté, dans ce qui suit, les éléments se rapportant à l'accessoire d'implantation 11 sont décrits par des références impaires tandis que les éléments se rapportant à la sonde 14 sont décrits avec des références paires.

La **Figure 1b** représentant une vue schématique et partielle de la sonde 14 flexible implantable de stimulation illustrée à la Figure 1a dans un état non implanté de la sonde, la Figure 1a et la Figure 1b sont décrits conjointement ci-après.

La sonde 14 comprend un corps de sonde 16.

La sonde 14 est apte à être combinée à un dispositif médical implantable actif 18 via l'extrémité proximale 20 de la sonde 14.

La sonde 14 est configurée pour être implantée via l'extrémité distale 22 de la sonde 14 à travers une paroi du coeur, en particulier à travers la paroi libre du ventricule droit. L'extrémité distale 22 est opposée à l'extrémité proximale 20 de la sonde 14.

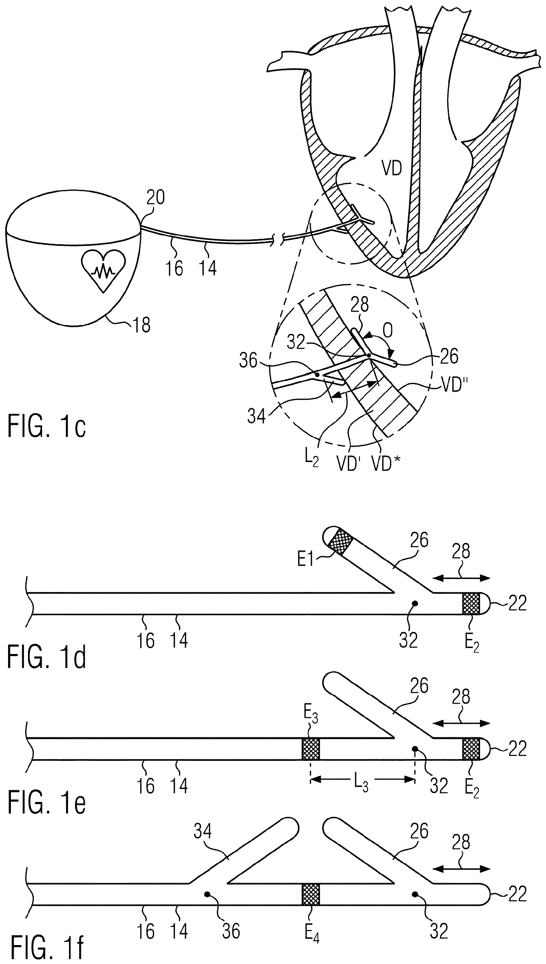

La **Figure 1c** illustre une vue schématique de la sonde 14 flexible implantable de stimulation dans un état implanté de la sonde.

Les éléments avec les mêmes références numériques utilisées pour la description de la Figure 1a, de la Figure 1b et de la Figure 1c se rapportent aux mêmes éléments et ne seront pas décrits à nouveau en détail pour chacune des Figures 1a, 1b et 1c.

La Figure 1a illustre une vue partielle d'une aiguille 13 comprise dans l'accessoire d'implantation 11 selon la présente invention.

L'aiguille 13 est pourvue d'une extrémité 15 libre pointue de ponction. L'extrémité 15 libre pointue de ponction correspond à l'extrémité distale de l'aiguille 13.

L'aiguille 13 est au moins dans sa partie distale, c'est-à-dire au moins la partie de l'aiguille 13 qui est illustrée à la Figure 1a, une aiguille creuse comprenant une lumière interne 17 débouchante sur l'extrémité 15 libre pointue de ponction.

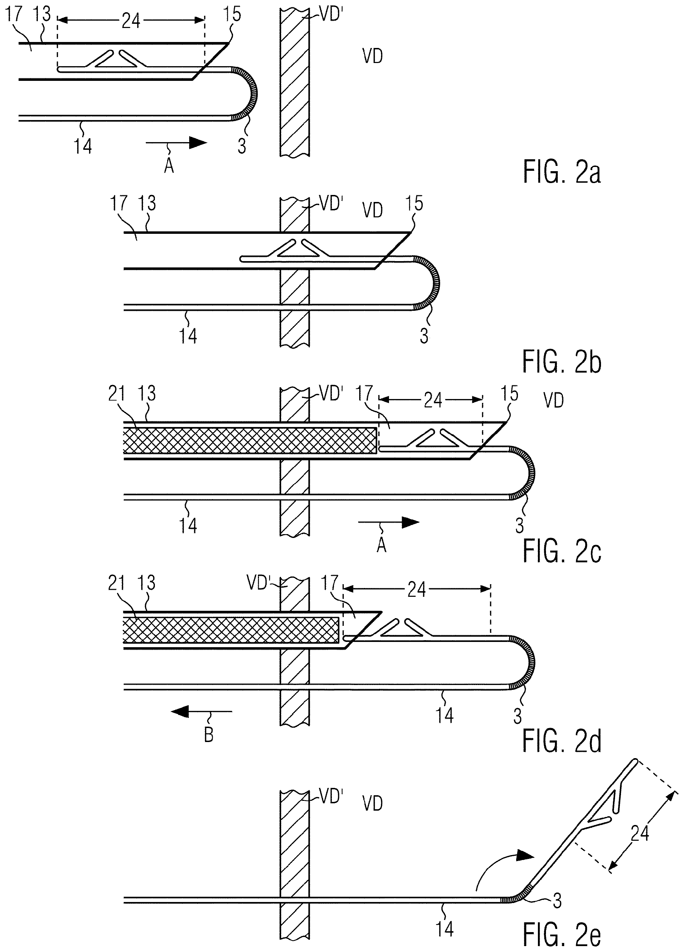

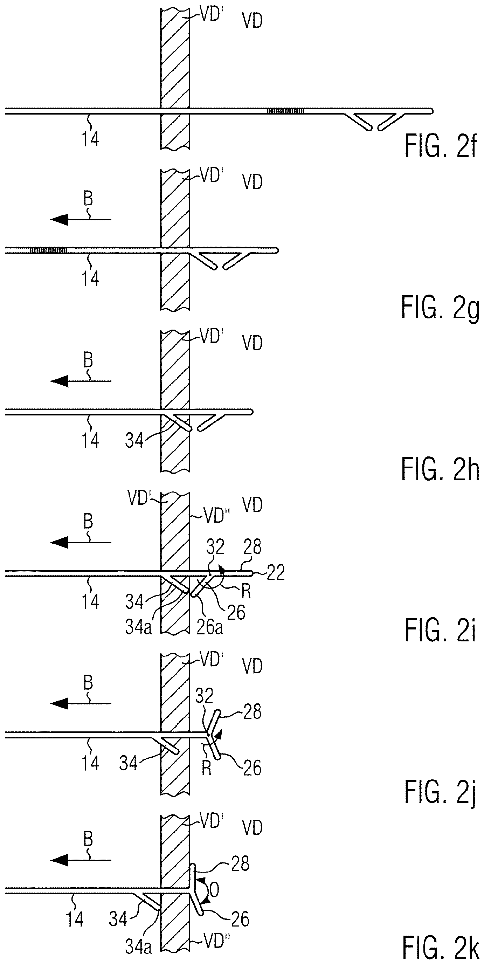

L'accessoire d'implantation 11 selon la présente invention comprend également un moyen poussoir apte à être logé et à coulisser dans la lumière 17 de l'aiguille 13 (ce moyen poussoir n'est pas représenté sur les Figures 1a à 1c). Ce moyen poussoir est visible sur les Figures 2c et 2d, qui seront décrites ci-après plus en détail.

Selon la présente invention, une première portion 24 de la sonde 14 est apte à être insérée via son extrémité distale 22 dans la lumière interne 17 de l'aiguille 13.

La Figure 1a illustre un état de la sonde 14 dans lequel la première portion 24 de la sonde 14 est insérée dans la lumière 17 de l'aiguille 13.

Notons que la hauteur h illustrée sur la Figure 1a entre l'aiguille 13 et le corps de sonde 16 qui n'est pas inséré dans la lumière 17 de l'aiguille 13 est représentée comme étant non nulle uniquement pas soucis de clarté pour l'illustration. L'homme du métier doit comprendre que pour la mise en oeuvre de la présente invention, à l'état dans lequel la première portion 24 de la sonde 14 est insérée dans la lumière 17 de l'aiguille 13, le reste du corps de sonde 16 s'étend le long de la paroi externe 19 de l'aiguille 13, de sorte qu'il y ait un contact possible, mais non nécessaire, entre le corps de sonde 16 et la paroi externe 19 de l'aiguille 13.

La première portion 24 de la sonde 14 comprend au moins une première branche 26 qui s'étend à partir du corps de sonde 16 dans une direction D orientée vers l'extrémité 15 libre pointue de l'aiguille.

La première branche 26 s'étend à partir du corps de sonde 16 depuis une distance prédéterminée L1 de l'extrémité distale 22 de la sonde 14. La portion du corps de sonde 16 de longueur L1 à partir de l'extrémité distale 22 forme une deuxième portion 28 du corps de sonde 16 disposée entre la première branche 26 et l'extrémité distale 22 de la sonde 14.

Tel qu'illustré à la Figue 1c, la première branche 26 et la deuxième portion 28 de la sonde 14 servent de moyen de retenue pour maintenir la sonde 14 à travers une paroi libre VD' du ventricule droit VD. Dans l'état implanté de la sonde 14 représenté à la Figure 1c, la première branche 26 et la deuxième portion 28 de la sonde 14 sont disposées de manière à s'étendre longitudinalement le long de la paroi interne VD" du ventricule droit VD.

Tel qu'illustré à la Figure 1b, la première branche 26 a une longueur l1 entre un point de jonction 32 du corps de sonde 16 de la première branche 26 et l'extrémité libre 26a de la première branche. La longueur l1 est comprise entre 2 et 20 mm.

La longueur L1 de la deuxième portion 28 et la longueur l1 de la première branche 26 peuvent être sensiblement égales entre elles.

Ces dimensions permettent d'améliorer la surface de contact du moyen de retenue contre la paroi interne VD" du ventricule droit VD et ainsi d'augmenter davantage la retenue de la sonde 14 à travers la paroi interne VD' du ventricule droit VD". De plus, la rigidité du moyen de retenue à proximité immédiate du point de jonction 32 permet de générer la rétention.

Dans un mode de réalisation de la présente invention tel que celui illustré aux Figures 1a à 1c, le corps de sonde 16 peut comprendre en outre une troisième portion 30 qui est moins rigide que la première branche 26 et la deuxième portion 28. La troisième portion 30 s'étend vers l'extrémité proximale 20 de la sonde 14, c'est-à-dire vers l'extrémité 15 libre pointue de l'aiguille dans la représentation de la Figure 1a. La troisième portion 30 s'étend depuis un point de jonction 32 du corps de sonde 16 entre la première branche 26 et la deuxième portion 28, de manière à ce que la première branche 26 et la deuxième portion 28 sont configurées pour pivoter (annoté par la référence R sur les Figures 1a et 1b) solidairement l'une de l'autre par rapport au point de jonction 32 du corps de sonde 16. La longueur de la troisième portion 30 peut être adaptée selon l'épaisseur du muscle cardiaque de la région visée.

Ainsi, la première branche 26 et de la deuxième portion 28 sont rendues solidaires l'une à l'autre lors d'un mouvement de pivotement R autour du point de jonction 32. Ce pivotement R permet de disposer le moyen de retenue de la sonde 14 contre la paroi interne VD" du ventricule droit VD dans un état implanté de la sonde 14, tel qu'illustré à la Figure 1c.

Tel qu'illustré à la Figure 1a, qui représente un état où la première portion 24 de la sonde 14 est insérée dans la lumière 17 de l'aiguille 13, la première branche 26 et la deuxième portion 28 forment un angle obtus O tandis que la première branche 26 et la troisième portion 30 forment un angle aigu A. Un angle obtus est un angle dont la mesure en degrés est comprise entre 90° et 180°. En particulier, l'angle obtus O formé entre la première branche 26 et la deuxième portion 28 a une mesure en degrés d'environ 135°.

Tel que décrit dessus, la première branche 26 et la deuxième portion 28 de la sonde 14 étant configurées pour pivoter solidairement l'une de l'autre par rapport au point de jonction 32 du corps de sonde 16, l'angle obtus O entre la première branche 26 et la deuxième portion 28 de la sonde 14 est conservé dans l'état implanté de la sonde 14, tel qu'illustré à la Figure 1c.

L'angle obtus O entre la première branche 26 et la deuxième portion 28 de la sonde 14 permet de disposer la première branche 26 et la deuxième portion 28 le long de la paroi interne VD" du ventricule droit VD et ainsi de fournir un moyen de retenue à la sonde 14.

Dans un mode de réalisation de la présente invention tel que celui illustré aux Figures 1a à 1c, la première portion 26 de la sonde 14 peut comprendre en outre un moyen d'appui formé par une deuxième branche 34 qui s'étend à partir du corps de sonde 16 dans une direction d orientée vers l'extrémité distale 22 de la sonde 14. La deuxième branche 34 s'étend à partir du corps de sonde 16 depuis un point de jonction 36. Le point de jonction 36 est compris dans une portion du corps de sonde 16 qui est à la fois distincte de la deuxième portion 28 et de la première branche 26.

Dans le mode de réalisation illustré la Figure 1a, le point de jonction 36 est compris dans la troisième portion 30 de la sonde 14. Dans une variante, le point de jonction 36 est situé sur le corps de sonde 16 au-delà de la troisième portion 30.

La deuxième branche 34 de la sonde 14 fournit un moyen d'appui qui permet d'améliorer le maintien de la sonde 14 à la paroi VD' du ventricule droit VD en empêchant une migration involontaire de la sonde 14 dans le ventricule droit VD après implantation de la sonde 14 (voir Figure 1c). Le moyen d'appui 34 prend en effet appui contre la paroi externe libre VD* du ventricule droit VD dans l'état implanté de la sonde 14, tel qu'illustré à la Figure 1c.

Tel qu'illustré à la Figure 1b, la deuxième branche 34 a une longueur l2 entre un point de jonction 36 du corps de sonde 16 de la deuxième branche 34 et l'extrémité libre 34a de la deuxième branche 34. La longueur l2 est comprise entre 2 à 20 mm.

La longueur l1 de la première branche 26 et la longueur l2 de la deuxième branche 34 peuvent être sensiblement égales entre elles.

Le point de jonction 36 de la deuxième branche 34 avec le corps de sonde 14 est espacé d'une longueur L2 comprise entre 1 à 30 mm du point de jonction 32 de la première branche 26.

Ces dimensions correspondent essentiellement à l'épaisseur de la paroi cardiaque VD' au niveau du ventricule droit VD. Ainsi, la sonde 14 est structurellement configurée pour que la première branche 26 et la deuxième branche 34 fassent saillie depuis le corps de sonde 16 de part et d'autre de la paroi VD' du ventricule droit VD dans l'état implanté de la sonde 14, chacune des branches 26, 34 permettant de retenir la sonde 14 au muscle cardiaque.

La sonde 14 étant une sonde de stimulation, elle comprend au moins une électrode de stimulation.

Dans une variante, la sonde 14 peut également comprendre au moins une électrode de détection.

Les Figures 1d à 1f illustrent des variantes de réalisation de première portion 24 de la sonde 14 comprenant au moins une électrode. Ces variantes sont combinables entre elles. La présente invention n'est pas limitée aux variantes illustrées aux Figures 1d à 1f.

Les éléments avec les mêmes références numériques déjà utilisées pour la description des Figures 1a à 1c ne seront pas décrits à nouveau en détail, et référence est faite à leurs descriptions ci-dessus.

La **Figure 1d** illustre une première portion 24 qui ne comprend qu'une seule branche 26. Dans cette variante, la première branche 26 et l'extrémité distale 22 de la deuxième portion 28 comprennent chacune une électrode E1, E2.

Comme la Figure 1d, la **Figure 1e** illustre une première portion 24 qui ne comprend qu'une seule branche 26. Dans cette variante, l'extrémité distale 22 de la deuxième portion 28 comprend une électrode E2 et le corps de sonde 16 comprend en outre une électrode E3 espacée d'une longueur L3 du point de jonction 32 de la sonde 14. La longueur L3 est comprise entre 2 et 50 mm, en particulier entre 2 et 30 mm.

L'électrode E3 peut être une cathode E3 qui est préférentiellement localisée côté proximal du point de jonction 32 de la sonde 14 afin de positionner la cathode E3 dans l'épaisseur du muscle cardiaque. Ainsi, il y a contact de la cathode E3 avec la paroi de ventricule droit dans l'épaisseur de la paroi VD' plutôt qu'en surface de la paroi interne VD".

La longueur L3 est ainsi définie comme la distance entre le point de jonction 32 et l'électrode E3, de manière à maintenir la cathode E3 dans le muscle cardiaque, i.e. dans l'épaisseur de la paroi VD'. En effet, les contacts de surface étant plus assujettis à des micro mouvements du fait du battement cardiaque, il est préférable de positionner la cathode E3 dans l'épaisseur de la paroi du ventricule droit VD'.

Ainsi, ces dimensions permettent, dans un état implanté de la sonde, de disposer l'électrode E3 dans l'épaisseur de la paroi cardiaque, en particulier de la paroi libre du ventricule droit. De ce fait, la stimulation est d'autant plus améliorée.

La **Figure 1f** illustre une première portion 24 qui comprend une première branche 26 et une deuxième branche 34. Dans cette variante, le corps de sonde 16 comprend une électrode E4 disposée entre le point de jonction 32 de la première branche 26 et le point de jonction 36 de la deuxième branche 34.

Ainsi, l'électrode E4 peut être avantageusement disposée dans l'épaisseur de la paroi libre du ventricule droit, ce qui améliore la stimulation du ventricule droit.

Dans une variante, la fonction de retenue de la deuxième branche 34 peut être obtenue via une conformation élastique selon un rayon de courbure (3 à 30 mm de rayon) du corps de sonde 16 débutant au niveau de l'électrode E4. Les avantages de cette variante résident dans le fait de moins impacter le sac péricardique et d'orienter le corps de sonde 16 tangentiellement à la paroi externe du muscle, configuration moins sollicitante pour le corps de sonde 16 (résistance à la fatigue mécanique). Cette courbure peut remplacer ou compléter la fonction de retenue de la deuxième branche 34.

Dans une autre variante, la sonde 14 peut être dotée de quatre électrodes électriquement indépendantes afin d'optimiser le système de stimulation/détection pour une zone d'implantation donnée.

Les électrodes de stimulation étant en contact direct avec la paroi du coeur, il est aussi rendu possible de stimuler à des énergies plus basses.

La présente invention permet aussi d'éviter d'avoir à introduire une sonde dans la cavité du ventricule droit pour la stimulation du ventricule droit. L'implantation de la sonde est ainsi rendue moins invasive. La vasculature du patient implanté n'est ainsi pas altérée.

De plus, selon un mode de réalisation, la sonde peut être un microcâble flexible comprenant un coeur électriquement conducteur revêtu d'une couche électriquement isolante, et la au moins une électrode peut être formée par une zone dénudée du microcâble. Dans ce mode de réalisation, le diamètre du microcâble est d'au plus 1 French (0,33mm).

Ainsi, grâce aux dimensions du microcâble, il est possible de rendre l'implantation de la sonde encore moins invasive.

L'avantage prépondérant de cette technique réside en effet dans le côté micro invasif. Au vu du très faible diamètre de la sonde, il est possible d'utiliser des aiguilles de ponction de gauge 18 à 24 permettant une mise en place par ponction subxiphoïdienne (sans nécessité de réaliser une ouverture chirurgicale nécessitant des moyens de fermeture tels que la pose d'une ou plusieurs sutures). Outre une récupération plus rapide du patient, l'avantage de cette approche est une diminution des risques infectieux.

En outre, le corps de sonde 16 selon la présente invention peut comprendre une zone 3 renforcée et élastique par rapport au reste du corps de sonde 16. Cette zone 3 est distincte de la première portion 24 et correspond à une zone de pliure de la sonde 14, tel qu'illustré à la Figure 1a. Ainsi, la robustesse, et donc la durée de vie de la sonde 14, peuvent être augmentées malgré le pliage de la sonde à cette zone 3.

L'implantation de la sonde 14 est davantage décrit en référence à la description de la méthode pour implanter une telle sonde à travers une paroi du coeur, en particulier à travers la paroi libre du ventricule droit au moyen des Figures 2a à 2k.

Les éléments avec les mêmes références numériques déjà utilisées pour la description des Figures 1a à 1c ne seront pas décrits à nouveau en détail, et référence est faite à leurs descriptions ci-dessus.

A la première étape de la méthode d'implantation selon la présente invention tel qu'illustré à la **Figure 2a**, une première portion 24 de la sonde 14 est insérée dans la lumière 17 de l'aiguille 13. La sonde 14 est pliée au niveau de la zone de pliure 3 tel qu'à la Figure 1a précédemment décrite.

L'aiguille 13 est ponctionnée depuis la surface thoracique puis approchée de la paroi du ventricule droit VD' selon la direction A.

L'aiguille 13 est déplacée jusqu'au ce que l'extrémité 15 libre de ponction de l'aiguille 13 ponctionne la paroi du ventricule droit VD' tel qu'illustré à la **Figure 2b**.

De nombreux système de guidage/repérage peuvent être utilisés : échographie de surface ou transœsophagienne, stimulation, repérage anatomique de surface, injection de produit de contraste sous amplificateur de brillance. Cette liste n'est pas exhaustive.

Alors que l'extrémité 15 libre de ponction de l'aiguille 13 est située à l'intérieur du ventricule droit VD, un moyen poussoir 21 de l'accessoire d'implantation selon la présente invention pousse la première portion 24 de la sonde 14 selon la direction A hors de la lumière 17 de l'aiguille 13, tel qu'illustré à la **Figure 2c**.

La **Figure 2d** représente une étape suivante à laquelle la première portion 24 de la sonde 14 est quasiment intégralement hors de la lumière 17 de l'aiguille 13.

Le moyen poussoir 21 est alors rétracté selon une direction B opposée à la direction A et l'aiguille 13 est retirée de la paroi VD' selon la direction B.

Ainsi, à l'étape de la **Figure 2e**, la première portion 24 de la sonde 14 est hors de l'aiguille 13 et est dans le ventricule droit VD. La flexibilité et l'élasticité de la zone de pliure 3 entraîne le déploiement de la sonde 14 jusqu'à la configuration illustrée à la **Figure 2f**, qui correspond aussi à celle de la Figure 2b précédemment décrite.

Ensuite, tel qu'illustré aux **Figures 2g** puis **2h**, la sonde 14 est légèrement tractée dans la direction B. La direction B s'étend de l'intérieur du ventricule droit vers l'extérieur du coeur.

A l'étape de la **Figure 2i**, la deuxième branche 34 glisse à travers la paroi du ventricule droit VD' vers l'extérieur du coeur selon la direction B. L'orientation de la deuxième branche 24 dont l'extrémité libre 34a pointe vers l'extrémité distale 22 de la sonde 14 permet de faciliter le mouvement de retrait selon la direction B de la deuxième branche 24.

L'extrémité libre 26a de la première branche 26 vient buter contre la paroi interne VD" du ventricule droit VD. Cette butée entraîne le pivotement R de la première branche 26 et de la deuxième portion 28 de la sonde 14 par rapport au point de jonction 32 tel qu'illustré à la Figure 2i.

Tel qu'illustré à la **Figure 2j**, le pivotement R continue de manière à pivoter la première branche 26 et la deuxième portion 28 de la sonde 14 d'environ 90° par rapport à la position initiale dans l'aiguille 13 telle qu'illustrée à la Figure 2a.

En même temps que le pivotement de la première branche 26 et de la deuxième portion 28, la sonde 14 continue à être tirée dans la direction B jusqu'à ce que la première branche 26 et la deuxième portion 28 de la sonde 14 viennent en appui contre la paroi interne VD" du ventricule droit CD tel qu'illustré à la **Figure 2k**, limitant ainsi le pivotement à un angle d'essentiellement 90°.

L'angle obtus O formé entre la première branche 26 et la deuxième portion 28 permet d'empêcher un retrait involontaire de la sonde 14 dans la direction B en positionnant la première branche 26 et la deuxième portion 28 de la sonde 14 en appui contre la paroi interne VD" du ventricule droit CD. En effet, lorsque la sonde 14 est tirée dans le sens et la direction B, la première branche 26 et la deuxième portion 28 de la sonde 14 butent contre la paroi interne VD" du ventricule droit CD. Cette butée empêche un retrait involontaire de la sonde 14 du ventricule droit.

Le bon positionnement de la sonde 14 peut être confirmé par les performances électriques attestant du bon positionnement des électrodes ou encore par un contrôle échographie ou sous amplificateur de brillance.

La deuxième branche 34 sert de moyen d'appui, l'extrémité libre 34a de la deuxième branche 24 venant buter contre la paroi externe VD* du coeur.

Le moyen d'appui 34 permet améliorer le maintien de la sonde 14 à la paroi VD du coeur en empêchant une migration involontaire de la sonde 14 selon la direction A dans le ventricule VD après implantation de ladite sonde.

## Revendications

1. Ensemble d'un accessoire d'implantation et d'une sonde flexible implantable de stimulation,
ladite sonde (14) comprenant un corps de sonde (16), et
ladite sonde (14) étant apte à être combinée à un dispositif médical implantable actif via son extrémité proximale (20) et étant configurée pour être implantée à travers une paroi du coeur, en particulier à travers la paroi libre (VD') du ventricule droit (VD), via son extrémité distale (22),
l'accessoire d'implantation comprenant une aiguille (13) avec une extrémité (15) libre pointue de ponction,
l'aiguille (13) étant au moins dans sa partie distale une aiguille creuse comprenant une lumière (17) interne débouchant sur l'extrémité (15) libre pointue de ponction, au moins une première portion (24) de la sonde (14) étant apte à être insérée via son extrémité distale (22) dans la lumière (17) interne de l'aiguille (13),
dans lequel :
dans un état où la première portion (24) de la sonde (14) est insérée dans la lumière (17) de l'aiguille (13), ladite première portion (24) de la sonde (14) comprend au moins une première branche (26) qui s'étend à partir du corps de sonde (16) dans une direction (D) orientée vers l'extrémité (15) libre pointue de l'aiguille (13), la première branche (26) s'étendant à partir du corps de sonde (16) depuis un point de jonction (32) disposé à une distance prédéterminée (L1) de l'extrémité distale (22) formant une deuxième portion (28) du corps de sonde (16) entre le point de jonction (32) de la première branche (26) et l'extrémité distale (22) de la sonde (14) ; et
le corps de sonde (16) comprend en outre une troisième portion (30) moins rigide que ladite première branche (26) et la deuxième portion (28), la troisième portion (30) s'étendant vers l'extrémité proximale (20) de la sonde (14) depuis le point de j onction (32) de la première branche (26), de manière que la première branche (26) et la deuxième portion (28) sont configurées pour pivoter solidairement l'une de l'autre par rapport audit point de j onction (32) en réponse à l'extrémité libre (26a) de la première branche (26) venant buter contre la paroi interne du coeur, en particulier contre la paroi interne (VD") du ventricule droit (VD), lors d'un mouvement de retrait de la sonde (14) de l'intérieur du coeur, en particulier de l'intérieur du ventricule droit (VD), vers l'extérieur du coeur.

2. Ensemble selon la revendication 1, dans lequel, dans un état où la première portion (24) de la sonde (14) est insérée dans la lumière (17) de l'aiguille (13), la première branche (26) et la deuxième portion (28) forment un angle obtus (O) et la première branche (26) et la troisième portion (30) forment un angle aigu (A).

3. Ensemble selon l'une des revendications précédentes, dans lequel la première branche (26) ou/et la deuxième portion (28) comprend au moins une électrode (E1, E2).

4. Ensemble selon l'une des revendications précédentes, dans lequel le corps de sonde (16) comprend au moins une électrode (E3) qui est espacée du point de jonction (32) de la sonde (14) par une longueur (L3) comprise entre 2 et 50 mm, en particulier entre 2 et 30 mm.

5. Ensemble selon l'une des revendications précédentes, dans lequel la première branche (26) et la deuxième portion (28) ont chacune une longueur (11, L1) comprise entre 2 et 20 mm.

6. Ensemble selon l'une des revendications précédentes, dans lequel le corps de sonde (16) comprend une zone (3) renforcée et élastique par rapport au reste du corps de sonde (16), ladite zone (3) étant distincte de la première portion (28) et correspondant à une zone de pliure (3) de la sonde (14).

7. Ensemble selon l'une des revendications précédentes, dans lequel la première portion de la sonde (24) comprend en outre un moyen d'appui formé par une deuxième branche (34) qui s'étend à partir du corps de sonde (16) dans une direction (d) orientée vers l'extrémité distale (22) de la sonde (14),
la deuxième branche (34) s'étendant à partir du corps de sonde (16) depuis un point de jonction (36) du corps de sonde (16) à la fois distinct de ladite deuxième portion (28) et de la première branche (26).

8. Ensemble selon la revendication 7, dans lequel le point de jonction (36) de la deuxième branche (34) avec le corps de sonde (16) est espacé d'une distance (L2) comprise entre 1 à 30 mm du point de jonction (32) de la première branche (26).

9. Ensemble selon la revendication 7 ou 8, dans lequel le corps de sonde (16) comprend au moins une électrode (E4) disposée entre le point de jonction (32) de la première branche (26) et le point de jonction (36) de la deuxième branche (34).

10. Ensemble selon l'une des revendications précédentes, dans lequel la sonde (14) est un microcâble flexible comprenant un coeur électriquement conducteur revêtu d'une couche électriquement isolante, et la au moins une électrode (E1, E2, E3, E4) étant formée par une zone dénudée du microcâble et le diamètre du microcâble étant d'au plus 1 French (0,33mm).

11. Sonde flexible implantable de stimulation pour un ensemble selon l'une des revendications précédentes, la sonde (14) comprenant un corps de sonde (16), et
ladite sonde (14) étant apte à être combinée à un dispositif médical implantable actif via son extrémité proximale (20) et étant configurée pour être implantée à travers une paroi du coeur, en particulier à travers la paroi libre du ventricule droit, via son extrémité distale (22),
la sonde (14) comprenant une première portion (24) configurée pour être insérée dans une aiguille de l'ensemble selon l'une des revendications précédentes, la première portion (24) comprenant au moins une première branche (26) qui s'étend à partir du corps de sonde (16) dans une direction (D) orientée vers l'extrémité proximale (20) de la sonde (14) et vers l'extrémité (15) libre pointue de l'aiguille lorsque la première portion (24) est insérée dans l'aiguille, la première branche (26) s'étendant à partir du corps de sonde (16) depuis une distance prédéterminée (L1) de l'extrémité distale (22) formant une deuxième portion (28) du corps de sonde (16) entre la première branche (26) et l'extrémité distale (22) de la sonde (14),
dans lequel le corps de sonde (16) comprend en outre une troisième portion (30) moins rigide que ladite première branche (26) et la deuxième portion (28), la troisième portion (30) s'étendant vers l'extrémité proximale (20) de la sonde (14) depuis le point de jonction (32) de la première branche (26), de manière que la première branche (26) et la deuxième portion (28) sont configurées pour pivoter solidairement l'une de l'autre par rapport audit point de jonction (32) en réponse à l'extrémité libre (26a) de la première branche (26) venant buter contre la paroi interne du coeur, en particulier contre la paroi interne (VD") du ventricule droit (VD), lors d'un mouvement de retrait de la sonde (14) de l'intérieur du coeur, en particulier de l'intérieur du ventricule droit (VD), vers l'extérieur du coeur.

12. Sonde flexible implantable de stimulation selon la revendication 11, dont la première portion de la sonde (24) comprend en outre un moyen d'appui formé par une deuxième branche (34) qui s'étend à partir du corps de sonde (16) dans une direction (d) orientée vers l'extrémité distale (22) de la sonde (14),
la deuxième branche (34) s'étendant à partir du corps de sonde (16) depuis un point de jonction (36) du corps de sonde (16) à la fois distinct de ladite deuxième portion (28) et de la première branche (26).

## Patentansprüche

1. Anordnung aus einem Implantierzubehör und einer implantierbaren flexiblen Stimulationssonde,
wobei die Sonde (14) einen Sondenkörper (16) aufweist, und
wobei die Elektrode (14) über ihr proximales Ende (20) mit einer aktiven implantierbaren medizinischen Vorrichtung kombiniert werden kann und so konfiguriert ist, dass sie durch eine Wand des Herzens, insbesondere durch die freie Wand (VD') des rechten Ventrikels (VD), über ihr distales Ende (22) implantiert werden kann,
wobei das Implantierzubehör eine Nadel (13) mit einem freien spitzen Punktionsende (15) aufweist,
wobei die Nadel (13) zumindest an ihrem distalen Teil eine Hohlnadel ist, die ein inneres Lumen (17) aufweist, das zu dem freien spitzen Punktionsende (15) führt, wobei zumindest ein erster Abschnitt (24) der Sonde (14) über sein distales Ende (22) in das innere Lumen (17) der Nadel (13) eingeführt werden kann;
wobei:
in einem Zustand, in dem der erste Abschnitt (24) der Sonde (14) in das Lumen (17) der Nadel (13) eingeführt wird, der erste Abschnitt (24) der Sonde (14) mindestens einen ersten Zweig (26) aufweist, der sich von dem Sondenkörper (16) in einer Richtung (D) erstreckt, die auf das spitze freie Ende (15) der Nadel (13) ausgerichtet ist, wobei sich der erste Zweig (26) von dem Sondenkörper (16) bis zu einem Verbindungspunkt (32) erstreckt, der in einem vorbestimmten Abstand (L1) von dem distalen Ende (22) angeordnet ist, der einen zweiten Abschnitt (28) des Sondenkörpers (16) zwischen dem Verbindungspunkt (32) des ersten Zweiges (26) und dem distalen Ende (22) der Sonde (14) bildet; und
der Sondenkörper (16) ferner einen dritten Abschnitt (30) bildet, der weniger steif ist als der erste Zweig (26) und der zweiten Abschnitt (28), wobei sich der dritte Abschnitt (30) bis zum proximalen Ende (20) der Sonde (14) von dem Verbindungspunkt (32) des ersten Zweigs (26) erstreckt, so dass der erste Zweig (26) und der zweite Abschnitt (28) so ausgebildet sind, dass sie sich in Bezug auf den Verbindungspunkt (32) fest miteinander drehen als Reaktion auf ein Auftreffen des freien Endes (26a) des ersten Zweiges (26) gegen die Innenwand des Herzens, insbesondere gegen die Innenwand (VD") des rechten Ventrikels (VD), während einer Bewegung des Zurückziehens der Elektrode (14) aus dem Inneren des Herzens, insbesondere von der Innenseite des rechten Ventrikels (VD) in Richtung der Außenseite des Herzens.

2. Anordnung nach Anspruch 1, wobei in einem Zustand, in dem der erste Abschnitt (24) der Sonde (14) in das Lumen (17) der Nadel (13) eingeführt wird, der erste Zweig (26) und der zweite Abschnitt (28) einen stumpfen Winkel (O) bilden und der erste Zweig (26) und der dritte Abschnitt (30) einen spitzen Winkel (A) bilden.

3. Anordnung nach einem der vorhergehenden Ansprüche, wobei der erste Zweig (26) oder/und der zweite Abschnitt (28) mindestens eine Elektrode (E1, E2) umfassen.

4. Anordnung nach einem der vorhergehenden Ansprüche, wobei der Sondenkörper (16) mindestens eine Elektrode (E3) aufweist, die vom Verbindungspunkt (32) der Sonde (14) um eine Länge (L3) zwischen 2 und 50 mm, insbesondere zwischen 2 und 30 mm, beabstandet ist.

5. Anordnung nach einem der vorhergehenden Ansprüche, wobei der erste Zweig (26) und der zweite Abschnitt (28) jeweils eine Länge (I1, L1) zwischen 2 und 20 mm aufweisen.

6. Anordnung nach einem der vorhergehenden Ansprüche, wobei der Sondenkörper (16) einen verstärkten und elastischen Bereich (3) in Bezug auf den Rest des Sondenkörpers (16) aufweist, wobei der Bereich (3) von dem ersten Abschnitt (28) verschieden ist und einem Krümmungsbereich (3) der Sonde (14) entspricht.

7. Anordnung nach einem der vorhergehenden Ansprüche, wobei der erste Abschnitt der Sonde (24) ferner ein Stützmittel umfasst, das aus einem zweiten Zweig (34) gebildet ist, der sich von dem Sondenkörper (16) in einer Richtung (d) erstreckt, die zum distalen Ende (22) der Sonde (14) orientiert ist, wobei sich der zweite Zweig (34) von dem Sondenkörper (16) von einem Verbindungspunkt (36) des Sondenkörpers (16) erstreckt, der sich sowohl von dem zweiten Abschnitt (28) als auch von dem ersten Zweig (26) unterscheidet.

8. Anordnung nach Anspruch 7, wobei der Verbindungspunkt (36) des zweiten Zweigs (34) mit dem Sondenkörper (16) um einen Abstand (L2) zwischen 1 und 30 mm vom Verbindungspunkt (32) des ersten Zweigs (26) beabstandet ist.

9. Anordnung nach Anspruch 7 oder 8, wobei der Sondenkörper (16) mindestens eine Elektrode (E4) umfasst, die zwischen dem Verbindungspunkt (32) des ersten Zweigs (26) und dem Verbindungspunkt (36) des zweiten Zweigs (34) angeordnet ist.

10. Anordnung nach einem der vorhergehenden Ansprüche, wobei die Sonde (14) ein flexibles Mikrokabel ist, das einen elektrisch leitenden Kern aufweist, der mit einer elektrisch isolierenden Schicht beschichtet ist, und wobei die mindestens eine Elektrode (E1, E2, E3, E4) durch eine abisolierte Fläche des Mikrokabels gebildet wird und der Durchmesser des Mikrokabels nicht mehr als 1 French (0,33 mm) beträgt.

11. Flexible implantierbare Stimulationssonde für eine Anordnung nach einem der vorhergehenden Ansprüche, wobei die Sonde (14) einen Sondenkörper (16) aufweist, und
wobei die Sonde (14) über ihr proximales Ende (20) mit einer aktiven implantierbaren medizinischen Vorrichtung kombiniert werden kann und so konfiguriert ist, dass sie über ihr distales Ende (22) durch eine Wand des Herzens, insbesondere durch die freie Wand des rechten Ventrikels, implantiert werden kann,
wobei die Sonde (14) einen ersten Abschnitt (24) aufweist, der so konfiguriert ist, dass er in eine Nadel der Anordnung nach einem der vorhergehenden Ansprüche eingeführt werden kann, wobei der erste Abschnitt (24) mindestens einen ersten Zweig (26) aufweist, der sich von dem Sondenkörper (16) in einer Richtung (D) erstreckt, die zum proximalen Ende (20) der Sonde (14) und zu dem spitzen freien Ende (15) der Nadel ausgerichtet ist, wenn der erste Abschnitt (24) in die Nadel eingeführt wird, wobei sich der erste Zweig (26) von dem Sondenkörper (16) in einem vorgegebenen Abstand (L1) von dem distalen Ende (22) erstreckt und einen zweiten Abschnitt (28) des Sondenkörpers (16) zwischen dem ersten Zweig (26) und dem distalen Ende (22) der Sonde (14) bildet,
wobei der Sondenkörper (16) ferner einen dritten Abschnitt (30) aufweist, der weniger steif ist als der erste Zweig (26) und der zweiten Abschnitt (28), wobei sich der dritte Abschnitt (30) von dem Verbindungspunkt (32) des ersten Zweigs (26) bis zum proximalen Ende (20) der Sonde (14) erstreckt, so dass der erste Zweig (26) und der zweite Abschnitt (28) so konfiguriert sind, dass sie sich in Bezug auf den Verbindungspunkt (32) fest miteinander drehen als Reaktion auf ein Auftreffen des freien Endes (26a) des ersten Zweiges (26) auf die Innenwand des Herzens, insbesondere auf die Innenwand (VD") des rechten Ventrikels (VD), während einer Bewegung des Zurückziehens der Elektrode (14) aus dem Inneren des Herzens, insbesondere von der Innenseite des rechten Ventrikels (VD) in Richtung der Außenseite des Herzens.

12. Flexible implantierbare Stimulationssonde nach Anspruch 11, wobei der erste Abschnitt der Sonde (24) ferner ein Stützmittel umfasst, das aus einem zweiten Zweig (34) gebildet ist, der sich von dem Sondenkörper (16) in einer Richtung (d) erstreckt, die zum distalen Ende (22) der Sonde (14) ausgerichtet ist,
wobei sich der zweite Zweig (34) von dem Sondenkörper (16) von einem Verbindungspunkt (36) des Sondenkörpers (16) erstreckt, der sich sowohl von dem zweiten Abschnitt (28) als auch von dem ersten Zweig (26) unterscheidet.

## Claims

1. An assembly of an implanting accessory and of a flexible implantable stimulation lead, said lead (14) comprising a lead body (16), and
said lead (14) being able to be combined with an active implantable medical device via its proximal end (20) and being configured to be implanted through a heart wall, in particular through the free wall (VD') of the right ventricle (VD), via its distal end (22), the implanting accessory comprising a needle (13) with a pointed free puncturing end (15),
the needle (13) being, at least in its distal part, a hollow needle comprising an inner lumen (17) opening onto the pointed free puncturing end (15), at least a first portion (24) of the lead (14) being able to be inserted via its distal end (22) into the inner lumen (17) of the needle (13),
wherein :
in a state where the first portion (24) of the lead (14) is inserted into the lumen (17) of the needle (13), said first portion (24) of the lead (14) comprises at least a first branch (26) that extends from the lead body (16) in a direction (D) oriented toward the pointed free end (15) of the needle (13), the first branch (26) extending from the lead body (16) from a junction point (32) arranged at a predetermined distance (L1) from the distal end (22) that forms a second portion (28) of the lead body (16) between the junction point (32) of the first branch (26) and the distal end (22) of the lead (14) ; and
the lead body (16) further comprises a third portion (30) that is less rigid than said first branch (26) and the second portion (28), the third portion (30) extending toward the proximal end (20) of the lead (14) from the junction point (32) of the first branch (26), such that the first branch (26) and the second portion (28) are configured to pivot securely with one another relative to said junction point (32) in response to the free end (26a) of the first branch (26) coming to abut against the inner wall of the heart, in particular against the inner wall (VD") of the right ventricle (VD), during a removal movement of the lead (14) from the inside of the heart, in particular from the inside of the right ventricle (VD), toward the outside of the heart.

2. The assembly of claim 1, wherein, in a state where the first portion (24) of the lead (14) is inserted into the lumen (17) of the needle (13), the first branch (26) and the second portion (28) form an obtuse angle (O) and the first branch (26) and the third portion (30) form an acute angle (A).

3. The assembly of any one of the preceding claims, wherein the first branch (26) and/or the second portion (28) comprise at least one electrode (E1, E2).

4. The assembly of any one of the preceding claims, wherein the lead body (16) comprises at least one electrode (E3) that is spaced away from the junction point (32) of the lead (14) by a length (L3) of between 2 and 50 mm, in particular between 2 and 30 mm.

5. The assembly of any one of the preceding claims, wherein the first branch (26) and the second portion (28) each have a length (11, L1) of between 2 and 20 mm.

6. The assembly of any one of the preceding claims, wherein the lead body (16) comprises a zone (3) that is reinforced and elastic relative to the rest of the lead body (16), said zone (3) being different from the first portion (28) and corresponding to a bending zone (3) of the lead (14).

7. The assembly of any one of the preceding claims, wherein the first portion of the lead (24) further comprises a bearing means formed by a second branch (34) that extends from the lead body (16) in a direction (d) oriented toward the distal end (22) of the lead (14), the second branch (34) extending from the lead body (16) from a junction point (36) of the lead body (16) that is both different from said second portion (28) and from the first branch (26).

8. The assembly of claim 7, wherein the junction point (36) of the second branch (34) with the lead body (16) is spaced apart by a distance (L2) of between 1 and 30 mm from the junction point (32) of the first branch (26).

9. The assembly of claim 7 or claim 8, wherein the lead body (16) comprises at least one electrode (E4) arranged between the junction point (32) of the first branch (26) and the junction point (36) of the second branch (34).

10. The assembly of any one of the preceding claims, wherein the lead (14) is a flexible microwire comprising an electrically conductive core coated with an electrically insulating layer, and the at least one electrode (E1, E2, E3, E4) being formed by a stripped zone of the microwire and the diameter of the microwire being no more than 1 French (0.33 mm).

11. A flexible implantable stimulation lead for an assembly of any one of the preceding claims, the lead (14) comprising a lead body (16), and
said lead (14) being able to be combined with an active implantable medical device via its proximal end (20) and being configured to be implanted through a heart wall, in particular through the free wall of the right ventricle, via its distal end (22),
the lead (14) comprising a first portion (24) configured to be inserted into a needle of the assembly of any one of the preceding claims, the first portion (24) comprising at least a first branch (26) that extends from the lead body (16) in a direction (D) oriented toward the proximal end (20) of the lead (14) and toward the pointed free end (15) of the needle when the first portion (24) is inserted into the needle, the first branch (26) extending from the lead body (16) at a predetermined distance (L1) from the distal end (22) that forms a second portion (28) of the lead body (16) between the first branch (26) and the distal end (22) of the lead (14),
wherein the lead body (16) further comprises a third portion (30) that is less rigid than said first branch (26) and the second portion (28), the third portion (30) extending toward the proximal end (20) of the lead (14) from the junction point (32) of the first branch (26) such that the first branch (26) and the second portion (28) are configured to pivot securely with one another relative to said junction point (32) in response to the free end (26a) of the first branch (26) coming to abut against the inner wall of the heart, in particular against the inner wall (VD") of the right ventricle (VD), during a removal movement of the lead (14) from the inside of the heart, in particular from the inside of the right ventricle (VD), toward the outside of the heart.

12. The flexible implantable stimulation lead of claim 11, wherein the first portion of the lead (24) further comprises a bearing means formed by a second branch (34) that extends from the lead body (16) in a direction (d) oriented toward the distal end (22) of the lead (14),
the second branch (34) extending from the lead body (16) from a junction point (36) of the lead body (16) that is both different from said second portion (28) and from the first branch (26).
